# EUROPEAN PATENT APPLICATION

(11) **EP 4 091 542 A1**
(43) Date of publication of application: **23.11.2022**
(21) Application number: 22173944.4
(22) Date of filing: 18.05.2022
(51) Int. Cl.: A61B 5/145, A61B 5/1455, A61B 5/283, A61N 1/05, A61B 5/00

(54) **CATHETER WITH BLOOD O2/CO2 CONCENTRATION MEASUREMENT**

(30) Priority: 19.05.2021 US 202117324266
(71) Applicant: Biosense Webster (Israel) Ltd, Yokneam, 2066717 (IL)
(72) Inventor: GOVARI, Assaf, 2066717 Yokneam (IL)
(74) Representative: Carpmaels & Ransford LLP

(57) **Abstract**

A probe for insertion into an organ of a patient includes a medical device and an optical sensor. The medical device is fitted at a distal end of the probe and configured to perform one or both of electrophysiological (EP) sensing and ablation of tissue inside the organ. The optical sensor is configured to locally acquire an optical signal indicative of a concentration of at least one gas in blood in the organ.

## Description

### FIELD OF THE INVENTION

The present invention relates generally to medical probes, and particularly to blood gas concentration measurement in catheters.

### BACKGROUND OF THE INVENTION

Various invasive medical probes to measure O₂ and/or CO₂ concentrations of the patient blood were proposed in the patent literature. For example, U.S. Patent 3,814,081 describes optical measuring catheter for measuring the degree of oxygen saturation in the blood of a living body. The catheter utilizes an illuminating fiber optical system and a light receiving fiber optical system arranged closely along each other, the forward ends of which are adapted to be inserted together into an organ of the living body in which the blood is flowing. The degree of oxygen saturation in the blood is determined by light incident to the blood and reflected thereby so as to be received by the light receiving fiber optical system due to the fact that the absorption spectrum of Hb is different from that of HbO₂.

As another example, U.S. Patent 4,201,222 describes an optical catheter including an absorption chamber and distensible semipermeable diaphragm are disclosed for the simultaneous measurement of blood gases, blood pressure and pulse rate.

U.S. Patent Application Publication 2011/0105869 describes a durable tissue pH/pCO₂ and/or tissue oxygen sensitive probe of sufficient strength to withstand direct tissue pressures in vivo, the probe comprising one or more sensor chambers within a biocompatible, gas-permeable membrane containing together in a single chamber, or in separate chambers, respectively, a pH sensitive fluorophore from which pCO₂ level (s) are calculated when the fluorophore is excited and the resulting fluorescence is measured, and/or an oxygen sensitive phosphor solution producing oxygen quenchable phosphorescence when excited. Further provided is a tissue pH/pCO₂ and/or tissue oxygen detection and measurement system comprising the probe.

U.S. Patent 4,476,870 describes a fiber optic probe to be implanted in human body tissue for physiologic studies involving measurement and monitoring of the partial pressure of gaseous oxygen in the blood stream, which is coursing through a particular blood vessel in the body. The use of the probe is based on the principle of dye fluorescence oxygen quenching. Structurally the probe comprises two strands of plastic optical fiber ending in a section of porous polymer tubing serving as a jacket or envelope for the fibers. The tubing is packed with a suitable fluorescent light-excitable dye placed on a porous adsorptive particulate polymeric support. The tubing or jacket is usually made of a hydrophobic, gas-permeable commercial material.

### SUMMARY OF THE INVENTION

An embodiment of the present invention that is described hereafter provides a probe for insertion into an organ of a patient, the probe including a medical device and an optical sensor. The medical device is fitted at a distal end of the probe and configured to perform one or both of electrophysiological (EP) sensing and ablation of tissue inside the organ. The optical sensor is configured to locally acquire an optical signal indicative of a concentration of at least one gas in blood in the organ.

In some embodiments, the at least one gas includes one or both of oxygen and carbon dioxide.

In some embodiments, the optical sensor includes a fluorescent element sensitive to the at least one gas.

In an embodiment, the tissue is cardiac tissue.

There is additionally provided, in accordance with another embodiment of the present invention, a medical system including a probe, an electrooptical measurement unit, and a processor. The probe is configured for insertion into a blood vessel of a body of a patient, wherein the probe includes (i) a distal-end medical device, configured to perform at least one of electrophysiological (EP) sensing and ablation of tissue inside the body, and (ii) an optical fiber configured to guide an optical signal to interact with blood in the blood vessel, and to output the optical signal that interacted with the blood. The electrooptical measurement unit is configured to collect and measure the outputted optical signal. The processor is configured to estimate a concentration of at least one gas in the blood by analyzing the measured optical signal from the probe.

In some embodiments, the medical system further includes a ventilator, which is configured to ventilate the patient based on the estimated concentration of the at least one gas in the blood.

In some embodiments, the medical system further includes an optical sensor at a distal end of the optical fiber, the optical sensor configured to locally acquire signals indicative of the concentration of the at least one gas in the blood.

In an embodiment, the tissue is wall tissue of the blood vessel.

In another embodiment, the distal-end medical device includes one or more sensing electrodes.

In yet another embodiment, the distal-end medical device includes one or more ablation electrodes.

There is further provided, in accordance with another embodiment of the present invention, a method including inserting a probe into an organ of a patient. Using a medical device fitted at a distal end of the probe, one or both of electrophysiological (EP) sensing and ablation of tissue inside the organ are performed. Using an optical sensor fitted in the probe, an optical signal is locally acquired, that is indicative of a concentration of at least one gas in blood in the organ.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a system for cardiac electrophysiological (EP) diagnostics and/or ablation comprising a catheter fitted with an optical fiber to acquire blood O₂/CO₂ concentration indicative signals, in accordance with an embodiment of the present invention;
Fig. 2 is a schematic, pictorial illustration combined with a block diagram, of a blood O₂/CO₂ concentration detection subsystem comprised in the system of Fig. 1, in accordance with an embodiment of the present invention; and
Fig. 3 is a flow chart that schematically illustrates a method to ventilate a patient using the subsystem of Fig. 2, in accordance with an embodiment of the present invention.

### DETAILED DESCRIPTION OF EMBODIMENTS

### OVERVIEW

In some invasive probing procedures (e.g., a surgical catheterization) a patient is intubated while the intravenous procedure is performed. During a procedure, blood oxygen (O₂) and carbon dioxide (CO₂) gas concentrations of the intubated patient are measured, for example, in order for a ventilator to deliver the correct flowrate of intubated air.

The gold standard for assessing respiratory status of an intubated patient is arterial blood gas analysis, using, for example, a dedicated arterial catheter. Blood O₂ and CO₂ concentrations may be measured by other independent means, such as a patient pulse oximeter and/or from measurements of the air exhaled by the patient. However, a dedicated probe is often required in order to measure the O₂ and CO₂ concentrations locally, (e.g., inside a cardiac chamber), which both complicates such invasive probing procedures and makes them more costly.

For example, a bedside arterial blood gas (ABG) monitoring system was reported, which uses fluorescent optical sensors in the measurement of blood pH, PCO₂ and PO₂. The Point-of-Care ABG system consists of the SensiCath^{™} optical sensor unit manufactured by Optical Sensors. As another example, a system for the continuous real-time, intra-arterial monitoring of arterial blood gas and acid base status (ABG) is the Optex BioSentry System, Optex Biomedical, Incorporated, The Woodlands, Texas, U.S.A. The system consists of three separate fiber optic channels with contained fluorescent and absorbant chemical analytes imbedded in a single probe and capable of insertion inside of a twenty gauge indwelling arterial catheter (The Optex Optode Sensor), along with an external monitor.

As yet a further example, Davenport et al. report in a paper, titled, " Dual pO2/pCO fiber optic sensing film," in The Analyst, 142(10), pp. 1711-1719, of a fiber optic multi-sensor for biomedical sensing applications using a tip coating solution sensitive to both oxygen and carbon dioxide. An oxygen sensitive phosphorescence quenching complex based on platinum octaethylporphyrin (PtOEP) was combined with a carbon dioxide sensitive phosphorescence compound based on 8-hydroxypyrene-1,3,6-trisulfonic acid trisodium salt (HPTS). When excited by blue light (470 nm), the resultant coating had two fluorescent peaks at 515 nm (green) and 645 nm (red) which responded to partial pressure of CO₂ and O₂ respectively. The sensor was tested in vitro and shown to be able to measure CO₂ and O₂ simultaneously and in real time. No significant cross-sensitivity or overlap was seen in O₂ and CO₂ measurements.

Embodiments of the present invention that are described hereinafter provide probes (e.g., catheters) that, in addition to performing diagnostics and/or treatment of tissue inside the body, include means to locally measure at least one gas concentration in the patient's blood, such as O₂ and/or CO₂ concentrations. Thus, while the probes perform their intended task (e.g., cardiac mapping and/or ablation), they additionally function as a dedicated probe for measuring O₂ and/or CO₂ concentrations.

Examples of probes that may incorporate means for locally measuring O₂ and/or CO₂ concentrations using the disclosed technique include focal catheters, as well as catheters having multiple electrodes on an expandable frame. However, the disclosed technique may be implemented, *mutatis mutandis*, with other invasive tools.

In an embodiment, a probe is provided that comprises a medical device, fitted at a distal end of the probe and configured to perform one or both of electrophysiological (EP) sensing and ablation of tissue inside the organ. The distal end further comprises an optical sensor configured to locally acquire an optical signal indicative of a concentration of at least one gas in blood in the organ.

In an example embodiment, a distal-end medical device such as of a coronary sinus (CS) catheter, which is regularly used for cardiac sensing and pacing during electrophysiological (EP) mapping examinations, further includes a fiber optic for measuring O₂ and/or CO₂ concentrations in the patient's cardiac blood. The tissue may be a wall tissue of the blood vessel or of a cardiac chamber.

The aforementioned means to locally measure (e.g., acquire signals indicative of blood O₂ and/or CO₂ concentrations may be commercially available technologies, such as optical (e.g., using a fiber optic), opto-chemical (e.g., using a distal sensor), among other potentially commercially available techniques. Accordingly, the probe may be proximally coupled to an external analyzer which is electrooptical, or by some other means, to analyze the signals indicative of O₂ and/or CO₂ concentration.

In one embodiment, the disclosed probe incorporates an optical fiber, with the fiber distal edge transmitting and collecting return light into and from a distally optically coupled O₂ and/or CO₂ sensitive fluorescent element that is in fluid contact with blood surrounding a distal end of the probe. The fiber optic is coupled, at a proximal end of the fiber, to the external optical O₂/CO₂ analyzer. The analyzer transmits light into the fiber optic, and determines, from an analysis of returned light (e.g., reflected back into the fiber optic), local O₂ and/or CO₂ blood concentrations.

In another embodiment, an opto-chemical sensor is incorporated at the fiber distal end, and opto-chemical signals, such as fluorescence, are transmitted from the sensor proximally for analysis by the external electrooptical unit.

By incorporating blood O₂/CO₂ concentration detection capability in a catheter configured to perform other tasks, the disclosed technique may simplify catheter based (e.g., cardiac) diagnostics and treatments.

### SYSTEM DESCRIPTION

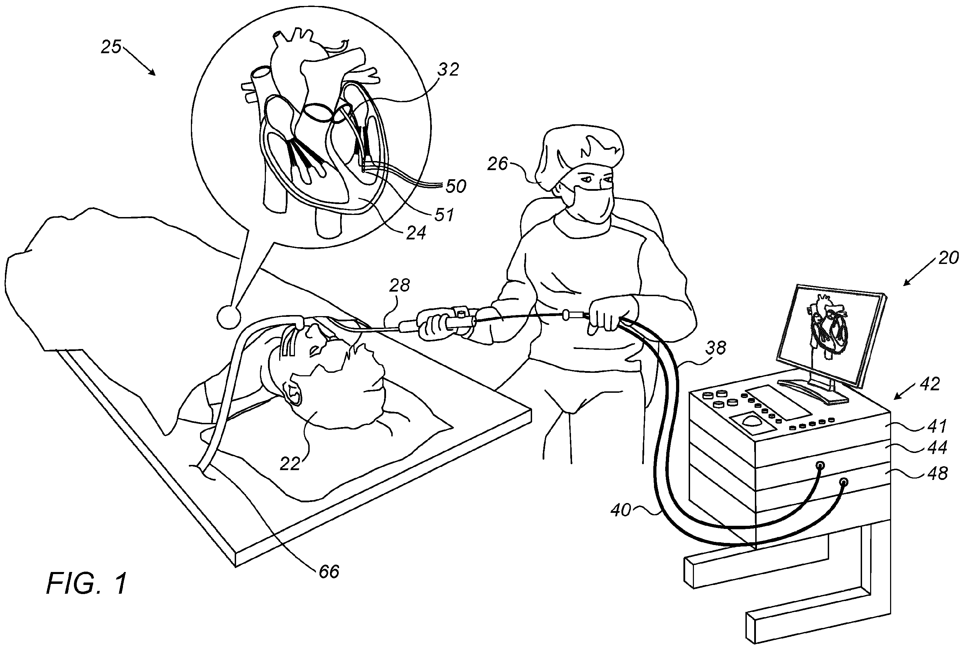

Fig. 1 is a schematic, pictorial illustration of a system 20 for cardiac electrophysiological (EP) diagnostics and/or ablation comprising a catheter 28 fitted with a fiber optic to acquire blood O₂/CO₂ concentration indicative signals, in accordance with an embodiment of the present invention. A distal end 32 of catheter 28, seen in inset 25, comprises one or more electrodes 50 and an optical fiber 40 having a cleaved edge 51 to transmit and collect return light to a distally optically coupled blood PO₂ and PCO₂ sensitive fluorescent element 515 that is in fluid contact with surrounding blood.

A physician 26 inserts catheter 28 through a blood vessel into a chamber of a heart 24 of a patient 22, and manipulates the catheter such that distal end 32 of the catheter accesses a cardiac chamber to be diagnosed/treated. As seen, patient 22 is intubated throughout the intravenous cardiac procedure, using an intubation tube 66.

In some embodiments, using a position-tracking system, a processor 41 accurately determines position coordinates of electrodes 50 of the catheter inside heart 26. In the shown embodiment, processor 41 determines the position coordinates based on measured impedances between the electrodes (on the catheter) and body surface electrodes (not shown). The method of electrode position sensing using system 20 is implemented in various medical applications, for example in the CARTO^{™} system, produced by Biosense Webster Inc. (Irvine, California) and is described in detail in U.S. Patents 7,756,576, 7,869,865, and 7,848,787, whose disclosures are all incorporated herein by reference.

After positioning distal end 32 in the cardiac chamber, physician 26 may, using electrodes 50, acquire EP signals and/or apply ablative energy (e.g., RF) to cardiac tissue via an electrical cable 38, using an ablation generator 44 in a console 42 to perform ablation.

A proximal end of fiber optic 40 is coupled to an optical O₂/CO₂ analyzer 48 in console 42. The analyzer (typically an electrooptical measurement unit) projects analyzing light into the fiber optic, and from an analysis of light returned (e.g., reflected) back into the fiber optic and outputted at the proximal end of the fiber, determines O₂ and/or CO₂ concentrations in the cardiac chamber's blood using, in the shown example, an algorithm run by processor 41.

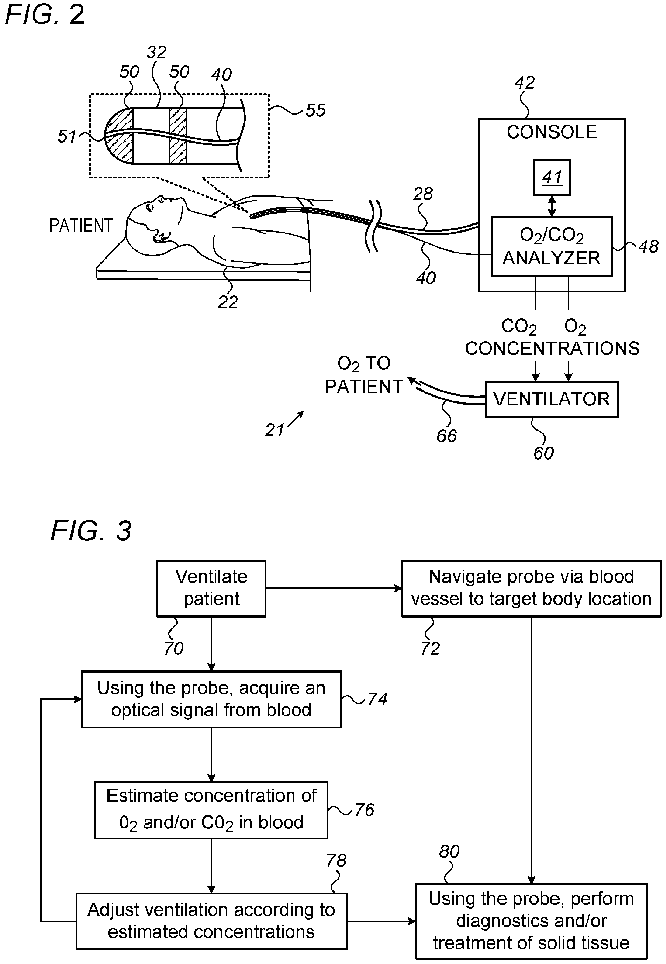

Processor 41 is typically programmed in software to carry out the functions described herein. The software may be downloaded to the computer in electronic form, over a network, for example, or it may, alternatively or additionally, be provided and/or stored on non-transitory tangible media, such as magnetic, optical, or electronic memory. In particular, processor 41 runs dedicated algorithms that enable processor 41 to perform the steps shown in Fig. 3.

Although the pictured embodiment specifically describes the use of a focal catheter comprising a single RF ablation electrode tip, the technique described herein may be applied with various kinds of probes and invasive tools, as described above.

### ADDING BLOOD O₂/CO₂ CONCENTRATION ACQUISITION TO CATHETER

Fig. 2 is a schematic, pictorial illustration, combined with a block diagram, of a blood O₂/CO₂ concentration detection subsystem 21 comprised in system 20 of Fig. 1, in accordance with an embodiment of the present invention.

As seen in inset 55, distal end 32 of catheter 28, which, by way of example, is a focal cardiac catheter comprising electrodes 50, includes optical fiber 40 with a fluorescent distal element 515 that is PO₂ and/or PCO₂ liquid-sensitive, to detect O₂ and CO₂ concentrations in blood. A cleaved edge 51 of fiber 40 enables the transmission of light to blood O₂ and/or CO₂ sensitive fluorescent element 515 and couples return fluorescence light into the fiber.

The proximal end of fiber optic 40 is coupled to a commercially available O₂/CO₂ electrooptical analyzer 48, that typically measures fluorescence signals to estimate partial pressure of O₂/CO₂. The analyzer transmits analyzing light into the fiber optic, and receives returned (e.g., reflected) light back into the fiber optic and outputted at the proximal end of the fiber. The analyzer analyzes the light (e.g., its level of attenuation or a change in its spectrum) to estimate concentrations of O₂ and CO₂ in the cardiac chamber blood. Normal blood gas concentrations for adults, given in partial pressure values, are 80-100mm Hg of pO₂ and 35-45 mm Hg of PCO₂. Mild to severe Hypoxemia is characterized by less than 80mm Hg of pO₂ and down to less than 40mm Hg of pO₂. Respiratory acidosis is characterized by over a 10 mmHg rise in pO₂. Respiratory alkalosis is characterized by over a 10 mmHg fall in pO₂. In the shown example, required computations are performed using a commercially available algorithm run by processor 41 to analyze fluoresce light from element 515.

The estimated blood O₂ and/or CO₂ concentrations are inputted to a processor of ventilator 60, which adjusts, during the intubation, ventilation power according to the concentrations.

Fig. 2 shows only elements related to the disclosed techniques for the sake of simplicity and clarity. For example, additional equipment, such as for anesthetizing, is not depicted.

Fig. 3 is a flow chart that schematically illustrates a method to ventilate a patient using subsystem 21 of Fig. 2, in accordance with an embodiment of the present invention. The algorithm, according to the presented embodiment, carries out a process that begins when patient 22 is intubated and ventilated, at a patient ventilation step 70.

Once the patient is ventilated, physician 26 navigates a probe, such as catheter 28, via blood vessels to a target location in the body of patient 22, at a probe navigation step 70.

As seen in Fig. 3, steps 74-78 take place continuously throughout the procedure, i.e., in parallel to steps 70-72. Moreover, step 80 may depend on an input from steps 74-78 that would indicate that the patient is in a stable respiratory state.

At an optical signal acquisition step 74, the probe acquires an optical signal indicative of concentration of O₂ and/or CO₂ in the blood.

In blood O₂/CO₂ concentrations estimation step 76, an optical sensing system analyzes the acquired optical signal to estimate these concentrations. As described above, one way to detect blood O₂/CO₂ concentrations is to illuminated and collect fluorescence from fluorescent element 515 in fluid communications with surrounding blood.

At a ventilation adjustment step 78, ventilator 60 adjusts the ventilation of patient 22 according to the estimated blood O₂/CO₂ concentrations. The process returns to step 74 to acquire new optical signals.

Finally, physician 26 operates the probe to perform diagnostics and/or treatment of solid tissue at the target location, at a diagnostic and/or treatment step 80.

The example flow chart shown in Fig. 3 is chosen purely for the sake of conceptual clarity. Additional steps may be included, such as inserting other invasive medical devices.

Although the embodiments described herein mainly address cardiac applications, the methods and systems described herein can also be used in other medical applications, such as in neurology and renal denervation.

It will be thus appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. A probe for insertion into an organ of a patient, the probe comprising:
a medical device, fitted at a distal end of the probe and configured to perform one or both of electrophysiological (EP) sensing and ablation of tissue inside the organ; and
an optical sensor configured to locally acquire an optical signal indicative of a concentration of at least one gas in blood in the organ.

2. The probe according to claim 1, wherein the at least one gas comprises one or both of oxygen and carbon dioxide.

3. The probe according to claim 1, wherein the optical sensor comprises a fluorescent element sensitive to the at least one gas.

4. The probe according to claim 1, wherein the tissue is cardiac tissue.

5. A medical system, comprising:
a probe for insertion into a blood vessel of a body of a patient, the probe comprising:
a distal-end medical device, configured to perform at least one of electrophysiological (EP) sensing and ablation of tissue inside the body; and
an optical fiber configured to guide an optical signal to interact with blood in the blood vessel, and to output the optical signal that interacted with the blood;
an electrooptical measurement unit, configured to collect and measure the outputted optical signal; and
a processor, configured to estimate a concentration of at least one gas in the blood by analyzing the measured optical signal from the probe.

6. The medical system according to claim 5, wherein the at least one gas comprises one or both of oxygen and carbon dioxide.

7. The medical system according to claim 5, and comprising a ventilator, which is configured to ventilate the patient based on the estimated concentration of the at least one gas in the blood.

8. The medical system according to claim 5, wherein an edge of the optical fiber is coupled to a fluorescent element sensitive to the at least one gas.

9. The medical system according to claim 5, and comprising an optical sensor at a distal end of the optical fiber, the optical sensor configured to locally acquire signals indicative of the concentration of the at least one gas in the blood.

10. The medical system according to claim 5, wherein the tissue is cardiac tissue.

11. The medical system according to claim 5, wherein the tissue is wall tissue of the blood vessel.

12. The medical system according to claim 5, wherein the distal-end medical device comprises one or more sensing electrodes.

13. The medical system according to claim 5, wherein the distal-end medical device comprises one or more ablation electrodes.

14. A method, comprising:
inserting a probe into an organ of a patient;
using a medical device fitted at a distal end of the probe, performing one or both of electrophysiological (EP) sensing and ablation of tissue inside the organ; and
using an optical sensor fitted in the probe, locally acquiring an optical signal indicative of a concentration of at least one gas in blood in the organ.
